# EUROPÄISCHE PATENTANMELDUNG

(11) **EP 1 480 151 A2**
(43) Veröffentlichungstag der Anmeldung: **24.11.2004**
(21) Anmeldenummer: 04010585.0
(22) Anmeldetag: 04.05.2004
(51) Int. Cl.: G06F 19/00

(54) **Verfahren zur Verarbeitung eines Therapiehinweise umfassenden Datensatzes bei medizinischen Behandlungen**

(30) Priorität: 20.05.2003 DE 10322685
(71) Anmelder: SIEMENS AKTIENGESELLSCHAFT, 80333 München (DE)
(72) Erfinder: Abraham-Fuchs, Klaus, 91058 Erlangen (DE); Rumpel, Eva Dr., 91052 Erlangen (DE); Tiffe, Sven, 1020 Wien (AT)

(57) **Zusammenfassung**

Die Erfindung betrifft ein Verfahren zur Verarbeitung eines Therapiehinweise umfassenden Datensatzes, insbesondere zur Qualitätssicherung bei medizinischen Behandlungen gemäß einem Therapiehinweis (1), wobei ein erster Datensatz (D1) eines Therapiehinweises (1) mindestens einen Eingang (2) und mindestens einen Ausgang (3) sowie eine den Eingang (2) und den Ausgang (3) verknüpfende Expertenregel (4) umfasst, wobei anhand der Eingangsdaten (E), welche dem Eingang (2) des betreffenden Datensatzes (D1) des zugrunde liegenden Therapiehinweises (1) zugeführt werden, mittels der Expertenregel (4) am Ausgang (3) des Datensatzes (D1) individuelle Therapiehinweise (Ti) in Form der Ausgangsdaten (A) erzeugt, ausgegeben und/oder hinterlegt werden.

## Beschreibung

Die Erfindung betrifft ein Verfahren zur Verarbeitung eines Therapiehinweise umfassenden Datensatzes zur Qualitätssicherung bei medizinischen Behandlungen gemäß einem Therapiehinweis. Solche Datensätze werden im Folgenden kurz selbst als Therapiehinweis bezeichnet. Die Datensätze, welche in einer zentralen Datenbank gespeichert sind, können sowohl Daten, also Therapiehinweise, als auch ausführbaren Code, also Softwarefragmente z. B. in Form von Expertenregeln, umfassen. Therapieinformationen sind Daten, Informationen und/oder Ressourcen zu einer diagnostischen und/oder therapeutischen Maßnahme bei einem Patienten oder einer Gruppe von Patienten.

In den letzten Jahren gewinnen zunehmend so genannte medizinische Leitlinien (im englischen Sprachraum als Medical Practise Guidelines" oder "Clinical Practise Guidelines"), kurz Leitlinie oder Guideline genannt, an Bedeutung. Leitlinien sind diagnostische und/oder therapeutische Handlungs- und Entscheidungsempfehlungen (im weiteren allgemein und zusammenfassend als Therapiehinweise bezeichnet) an den Arzt, die von übergeordneten und allgemein anerkannten Gremien der Ärzteschaft in einem breiten Konsens erarbeitet wurden. Naturgemäß müssen solche Leitlinien ständig dem neuesten Stand des Wissens angepasst werden, und unterliegen somit einem ständigen, wenn auch meist lang- oder mittelfristigen Wandel. Typischerweise werden Leitlinien in Intervallen von wenigen Jahren neu überarbeitet und angepasst. Dabei besteht stets das Bemühen, eine Behandlung von Patienten entsprechend einer Diagnose- oder Therapieempfehlung gemäß solcher etablierter Leitlinien ständig zu verbessern und somit Qualitätssicherungsmaßnahmen auch in der Medizin zu etablieren.

Hinsichtlich einer detaillierten Erläuterung von Therapiehinweisen und Therapieinformationen sowie deren Abhängigkeiten und Wechselwirkungen wird auf die am gleichen Tage vom selben Anmelder eingereichte Patentanmeldung mit dem Titel "Verfahren zur Referenzierung von Therapiehinweise umfassenden Datensätzen" verwiesen.

Im Stand der Technik sind nach bestem Wissen der Anmelderin bisher keine automatisch ablauffähigen Verfahren zur Sicherung der Qualität einzelner Therapieentscheidungen mittels Therapiehinweisen bekannt geworden.

Die Erfindung besteht damit darin, ein Verfahren zur Verarbeitung von Therapiehinweise umfassenden Datensätze anzugeben, mit dem in besonders einfacher Art und Weise eine automatische und sichere sowie qualitativ hochwertige individuelle Datenanalyse und/oder Datenausgabe für eine Therapieentscheidung ausgeführt werden kann.

Diese Aufgabe wird erfindungsgemäß mit den Merkmalen des Anspruchs 1 gelöst. Dazu ist bei einem Verfahren zur Verarbeitung eines Therapiehinweise umfassenden Datensatzes, insbesondere zur Qualitätssicherung bei medizinischen Behandlungen vorgesehen, dass der Datensatz eines Therapiehinweises mindestens einen Eingang und mindestens einen Ausgang sowie eine Ein- und Ausgang verknüpfende Expertenregel umfasst, und ein Therapieinformationen umfassender zweiter Datensatz mit Eingangsdaten des ersten Datensatzes des betreffenden Therapiehinweises derart verknüpft wird, dass die Eingangsdaten anhand für die medizinische Behandlung erforderlichen Parametern der Therapieinformation des zweiten Datensatzes bestimmt und dem ersten Datensatz zugeführt werden, wobei anhand der Eingangsdaten mittels der Expertenregel am Ausgang des ersten Datensatzes individuelle Therapiehinweise in Form der Ausgangsdaten erzeugt, ausgegeben und/oder hinterlegt werden.

In einer besonders vorteilhaften Weiterbildung wird dabei für eine Behandlung gemäß eines Therapiehinweises ein Therapieinformationen umfassender zweiter Datensatz mit den Eingangsdaten und/oder den Ausgangsdaten des ersten Datensatzes verknüpft. Beispielsweise werden für eine Verknüpfung von Therapiehinweisen, wie diagnostische und/oder therapeutische Handlungsempfehlungen mit Therapieinformationen, wie Patientendaten und/oder diagnostische und/oder therapeutische Mittel und Ressourcen vorzugsweise die Eingangsdaten für einen vorgegebenen oder betreffenden Therapiehinweis anhand von für die medizinische Behandlung erforderlichen Parametern, wie beispielsweise Messdaten, Erkenntnisse aus vorangegangenen Diagnosen oder bereits durchgeführte Therapien, der Therapieinformation bestimmt und dem ersten Datensatz des betreffenden Therapiehinweises zugeführt.

Die Erfindung geht von der Erkenntnis aus, dass parallel und unabhängig von der Einführung von Leitlinien in der Medizin derzeit eine starke Ausweitung des Einsatzes moderner Informations- und Kommunikationstechnologie im Gesundheitswesen stattfindet. Der Einsatz elektronischer Datenverarbeitung im Krankenhaus (z. B. HIS = Hospital Information System, RIS = Radiology Information System, PACS = Picture Archive & Communication System, LIS = Laboratory Information System) und in der Arztpraxis (Praxisverwaltungssoftware, elektronische Patientenakte) wird mehr und mehr üblich. Von einem folgenden Entwicklungsschritt wird allgemein erwartet, dass eine Vernetzung dieser Software und Datenbanken über die Institutionen des Gesundheitswesens (Kliniken, Arztpraxen, Therapeutenpraxen etc.) hinweg stattfinden wird. Damit wird die Möglichkeit für ein "Vernetztes Gesundheitswesen", zuerst auf nationaler oder regionaler Ebene und später global geschaffen. Diese Entwicklung bietet die Basis zum Einsatz der Erfindung.

Der Vorteil der Erfindung besteht darin, dass erstmals medizinisches Handeln anhand von individuellen Therapiehinweisen in Form von Ausgangsdaten, wie beispielsweise in Form von Text oder Bild, in einer automatisch auswertbaren Art und Weise generiert, weiterverarbeitet, ausgegeben und/oder dokumentiert wird sowie automatisch auf Konformität mit etablierten medizinischen Therapiehinweisen, insbesondere etablierten medizinischen Leitlinien, überprüft werden kann. Diese Überprüfung kann sich auf einzelne Mediziner, also Ärzte oder Therapeuten, einzelne medizinische Institutionen oder eine Gruppe von derartigen Institutionen usw. beziehen. Damit ist bei einer Vielfalt von Therapien zur Behandlung gleichartiger Krankheitsbilder die Behandlung solcher gleichartiger Krankheitsbilder über eine Vielzahl von Ärzten und Institutionen hinweg hinsichtlich der Konformität der einzelnen Therapiemaßnahmen mit etablierten Therapiehinweisen analysierbar.

Vorteilhafte Ausgestaltungen der Erfindung sind Gegenstand der Unteransprüche.

Zweckmäßigerweise werden die Eingangsdaten dem ersten Datensatz des Therapiehinweises ereignis- und/oder zeitgesteuert zugeführt. Auch ist es vorteilhaft, wenn die Ausgangsdaten automatisch oder ereignisbedingt ausgegeben werden. Beispielsweise wird bei einer Identifizierung von spezifischen Eingangsdaten einer Therapieinformation, wie bestimmte Laborwerte eines Patienten oder Diagnose eines Facharztes für einen Patienten, und somit ereignisgesteuert eine Zuführung dieser spezifischen Eingangsdaten an den Eingang des ersten Datensatzes erfolgen, so dass mittels der Expertenregel des ersten Datensatzes individuelle Therapiehinweise hergeleitet oder diese auf Konformität überprüft und anschließend ausgegeben werden können. Die Überprüfung auf Übereinstimmung eines individuellen Therapiehinweises oder einer Handlungsempfehlung bzw. -entscheidung anhand der Expertenregel erfolgt ereignisgesteuert beispielsweise bei jeder neuen Diagnosestellung oder jeder neuen Therapieentscheidung, nach jeder Überweisung an einen weiterbehandelnden Arzt oder nach jedem neuen Eintrag in eine Patientenakte bzw. zeitgesteuert beispielsweise periodisch mit beliebig wählbarem Zyklus oder nach jedem Aktualisierungszyklus. Dabei kann die Ausgabe automatisch oder ereignisbedingt ausgeführt werden. Beispielsweise kann die Ausgabe des individuellen Therapiehinweises nur im Falle einer Konformitätsverletzung oder nur im Falle einer Konformitätsbestätigung erfolgen.

Bevorzugt wird dem Therapiehinweis ein Ordnungsmerkmal zugeordnet, anhand dessen der Therapiehinweis eindeutig identifiziert wird. Zweckmäßigerweise wird dabei das Ordnungsmerkmal desjenigen Therapiehinweises, welcher der medizinischen Behandlung zugrunde liegt, der Therapieinformation zugeordnet. Hierdurch ist vorteilhaft, eine jederzeitige Zuordnung der Therapieinformation - z. B. eines Patienten - zum zugrunde liegenden Therapiehinweis - z. B. eine Diagnose- oder Therapieempfehlung - gewährleistet. Zusätzlich werden vorteilhafterweise dem Ordnungsmerkmal die Eingangsdaten und/oder die Ausgangsdaten des zugrunde liegenden Therapiehinweises zugeordnet.

Vorteilhaft wird der Therapieinformation mindestens ein optionales Eingangsdatum und/oder mindestens ein optionales Ausgangsdatum zugeordnet. Damit können in einer Therapieinformation auch solche Eingangs- und Ausgangswerte dauerhaft und nachvollziehbar gespeichert werden, die bei verketteten Therapiehinweisen als Eingangswerte für in einer solchen Verkettung nachfolgende Therapiehinweise oder als Ausgangswerte von solchen in einer Verkettung nachfolgenden Therapiehinweisen generiert werden. Zusätzlich oder alternativ wird der Therapieinformation mindestens ein weiteres frei definierbares Eingangsdatum und/oder mindestens ein weiteres frei definierbares Ausgangsdatum zugeordnet. Dies ermöglicht in besonders einfacher Form, zusätzlich zu den Mindestinformationen weitere Informationen, also Eingangs- und Ausgangswerte, die gemäß dem zugrunde liegenden Therapiehinweis nicht erforderlich oder nicht vorgesehen sind, dauerhaft und nachvollziehbar in der Patientenakte zu speichern.

Die Speicherung der Informationen erfolgt im Hinblick auf darauf basierenden Qualitätssicherungsmaßnahmen in besonders günstiger Art und Weise, wenn die vom Therapiehinweis an dessen mindestens einem Ausgang generierten individuellen Therapiehinweise automatisch in die jeweiligen Ausgangsdaten der zugehörigen Therapieinformation übertragen werden. Eine beabsichtigte oder unbeabsichtigte Verfälschung der gespeicherten Informationen ist damit wirksam verhindert.

In einer weiteren bevorzugten Ausführungsform wird eine manuelle Änderung einzelner der Therapieinformation zugeordneter Ausgangsdaten und/oder einzelner Eingangsdaten, insbesondere der frei definierbaren Eingangs- und Ausgangsdaten, gemäß einem vorgegebenen Format ausgeführt, so dass eine Grundlage für eine automatische Auswertung der in der Therapieinformation enthaltenen Informationen geschaffen ist.

Um die Sicherheit hinsichtlich beabsichtigter oder unbeabsichtigter Verfälschung der gespeicherten Informationen noch weiter zu erhöhen, ist vorteilhaft vorgesehen, dass bei oder vor einer Änderung einzelner Eingangs- oder Ausgangsdaten eine diesbezügliche Berechtigung überprüft wird.

Die Speicherung der Informationen, insbesondere der Eingangs- und Ausgangsdaten, ggf. inklusive der frei definierbaren Eingangs- und Ausgangsdaten und/oder der optionalen Ausgangsdaten, erfolgt vorteilhaft in einer dafür vorgesehenen Datenstruktur, die entsprechend der Abbildung der Informationen und Therapieanweisungen, die zu Beginn eines bestimmten Behandlungsstadiums, also vor einem Behandlungsschritt, vorliegen, kurz selbst als Behandlungsschritt bezeichnet wird. Jeder Therapiehinweis umfasst mindestens eine solche Datenstruktur, also mindestens einen Behandlungsschritt. Bei einer Mehrzahl von Behandlungsschritten für eine einzelne Therapieinformation werden diese vorteilhaft in Form einer als verkettete Liste realisierten Datenstruktur, die, nachdem sie die Behandlungsfolge beinhaltet, selbst kurz ebenfalls als Behandlungsfolge bezeichnet wird, zugeordnet. Bei einer grundsätzlich im vorhinein nicht bekannten Anzahl von Eingangs- und Ausgangsdaten sind diese vorteilhaft in Form von verkettete Listen darstellenden Datenstrukturen gespeichert.

Besonders vorteilhaft werden die in einzelnen oder in einer Mehrzahl von Therapieinformationen gespeicherten Eingangs- und Ausgangsdaten zur Ergänzung und/oder Aktualisierung von Therapiehinweisen verwendet. Damit ist eine Art von Regelkreis etabliert, wobei insbesondere aus einer Vielzahl von frei definierbaren Eingangs- und Ausgangswerten in einer Mehrzahl von Therapieinformationen, die sämtlich auf dem gleichen Therapiehinweis oder auf ähnlichen Therapiehinweisen basieren, ein Maß für die Notwendigkeit der Änderung der betroffenen Therapiehinweise abgeleitet wird. Mit der Änderung der Therapiehinweise, also deren Anpassung an die tatsächlichen Gegebenheiten und Bedürfnisse, variiert die Anzahl von frei definierbaren Eingangs- und Ausgangswerten in denjenigen Therapieinformationen, die auf den geänderten Therapiehinweisen basieren. Andere Mediziner profitieren bei der Verwendung der geänderten Therapiehinweise von deren besseren Ausrichtung auf die praktischen Anwendungsfälle.

Ein weiterer Vorteil der Erfindung und ihrer Ausgestaltungen besteht darin, dass fehlerhafte Behandlungen von Patienten schnell erkannt werden. Heute kann es vorkommen, dass Patienten über einen längeren Zeitraum fehlerhaft behandelt werden, weil zu einem bestimmten Zeitpunkt im Behandlungsprozess eine Diagnose fehlerhaft gestellt wurde und diese Diagnose in der Folgezeit nicht oder nicht ausreichend hinterfragt wird. Im Zuge einer Überweisung übernimmt ein Arzt die weitere Behandlung des Patienten und verlässt sich auf die in den Unterlagen des Patienten angegebene Diagnose. Diese Vorgehensweise ist durchaus sinnvoll, da sonst Mehrfachuntersuchungen in erheblichem Umfang mit der entsprechenden Kostenlast nötig wären. Hier schafft die Erfindung die Grundlage für ein mit geringem Aufwand ausführbares Verfahren, die ursprüngliche Diagnosestellung zu überprüfen, um so die Minderheit von mit einer falschen Diagnose behafteten Krankengeschichten identifizieren zu können. Nachdem sich auch die Kriterien für eine Diagnosestellung oder Therapieentscheidung im Laufe der Zeit ändern kann, etwa Grenzwerte angehoben oder abgesenkt werden oder völlig neue Therapien oder Technologien verfügbar sind, besteht auch die Möglichkeit, veraltete Diagnosestellungen oder Therapieentscheidungen mit der gleichen Effizienz zu erkennen.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen.

Darin zeigen:
- FIG 1: eine schematische Darstellung der softwaremäßigen Realisierung eines Therapiehinweises,
- FIG 2: einen Therapieinformationen umfassenden Datensatz,
- FIG 3: eine Variante der Therapieinformation,
- FIG 4: eine Therapieinformation mit frei definierbaren Eingangs- und Ausgangsdaten,
- FIG 5: eine Therapieinformation zur ausschließlichen Dokumentation von Ausnahmefällen,
- FIG 6: kaskadierte (verkettete) Therapiehinweise,
- FIG 7: eine Therapieinformation für verkettete Therapiehinweise und
- FIG 8: eine zentrale Datenbank mit Therapiehinweisen und eine lokale Datenbank mit Therapieinformationen, die auf einzelnen Therapiehinweisen der zentralen Datenbank basieren.

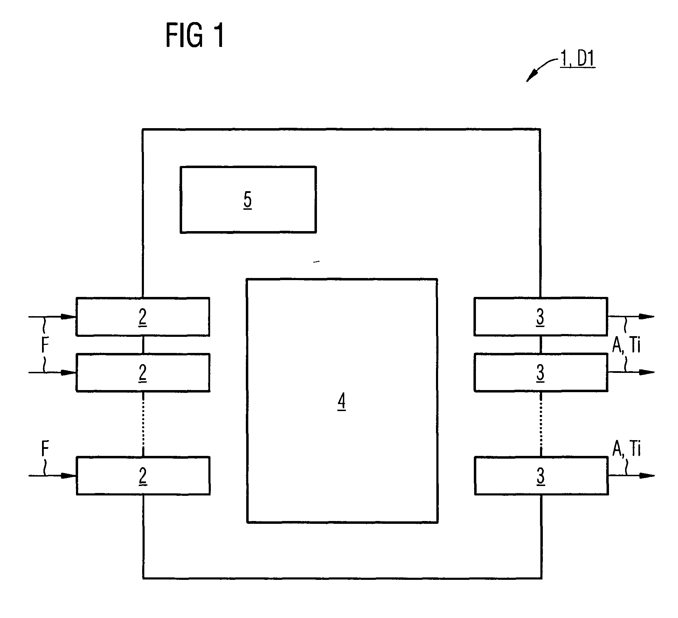

FIG 1 zeigt eine schematische Darstellung eines ersten Datensatzes D1 für die softwaremäßige Realisierung der Grundstruktur einer medizinischen Leitlinie, also eines Therapiehinweises 1. Der den Therapiehinweis 1 umfassende erste Datensatz D1 wird im weiteren kurz als Therapiehinweis bezeichnet.

Der Therapiehinweis 1 und somit der erste Datensatz D1 des Therapiehinweises 1 umfasst zumindest einen Eingang 2 und zumindest einen Ausgang 3. Den Kern des Therapiehinweises 1 bildet eine Expertenregel 4. Die Expertenregel 4 generiert aufgrund von Eingangsdaten E, z. B. von Informationen wie Patientendaten oder Informationen über diagnostische und/oder therapeutische Mittel an dem oder jedem Eingang 3 an einem oder mehreren Ausgängen 3 individuelle Therapiehinweise Ti in Form von Ausgangsdaten A. Die Informationen an einem Eingang 2 können von verschiedenen Kategorien, z. B. Messdaten wie Blutdruck, Elektrokardiogramm, etc., Erkenntnisse wie eine vorausgehende Diagnose oder Handlungen wie eine durchgeführte Therapieoption, etc. sein. Ebenso können die an den Ausgängen 3 ausgegebenen individuellen Therapiehinweise Ti von verschiedenen Kategorien sein, z. B. eine Erkenntnis wie eine aus den Eingangswerten oder -daten E abgeleitete Diagnose, eine Handlungsempfehlung oder aus den Eingangsdaten E abgeleitete weitere Daten. Die Expertenregel 4 ist im Allgemeinen eine so genannte Interferenz-Regel, mit der aus den Eingangsdaten E einzelne oder mehrere individuelle Therapiehinweise 1 abgeleitet werden.

Jeder Therapiehinweis 1 umfasst ein den Therapiehinweis 1 eindeutig kennzeichnendes Ordnungsmerkmal 5. Das Ordnungsmerkmal 5 ist geeignet und vorgesehen, einen Therapiehinweis 1, der z. B. in einer zentralen Datenbank (nicht dargestellt) gespeichert ist und in einer medizinischen Institution verwendet wird, zu referenzieren.

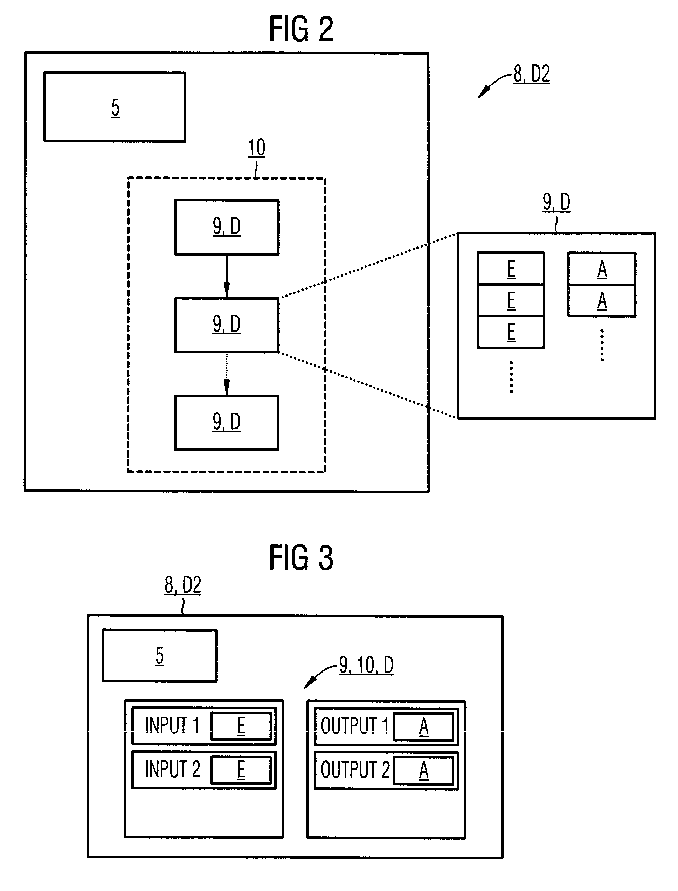

FIG 2 zeigt schematisch einen auf Seiten der medizinischen Institution, das heißt z. B. der Arztpraxis oder der Klinik, für jeden Patienten oder ggf. für eine Gruppe von Patienten geführten und Therapieinformationen 8 umfassenden zweiten Datensatz D2. Ein solcher Therapieinformationen 8 umfassender zweiter Datensatz D2 wird im Folgenden kurz auch als Therapieinformation 8 bezeichnet. Eine gebräuchliche Form einer Therapieinformation 8 ist eine elektronische Patientenakte. Die Therapieinformation 8 kann sich im Einzelfall auch auf räumlich verteilte Datenbanken erstrecken. Wichtig ist im Folgenden aber eher das Layout einer solchen Therapieinformation 8 und die jeweils hinterlegten und miteinander verknüpften Informationen und weniger der konkrete Speicherort einzelner oder sämtlicher Informationen oder Daten.

Jede Therapieinformation 8 umfasst das Ordnungsmerkmal 5 desjenigen Therapiehinweises 1 auf dem die Therapieinformation 8 basiert. Das heißt, jeder Patientenakte wird zur Dokumentation der medizinischen Behandlung des Patienten das Ordnungsmerkmal 5 des betreffenden Therapiehinweis 1 zugeordnet, insbesondere dort im zweiten Datensatz D2 hinterlegt. Zusammen mit dem Ordnungsmerkmal 5 sind bedarfsweise Daten über den Beginn, über die bisherige Dauer, über das voraussichtliche oder tatsächliche Ende der medizinischen Behandlung usw. gespeichert.

Zu genauen und sicheren Dokumentation der medizinischen Behandlung umfasst die Therapieinformation 8 Daten D zu einzelnen Behandlungsschritten 9, also zum einen Beschreibungen oder Auflistungen von Symptomen, Messwerten und dergleichen sowie zum anderen Medikationen und ähnliches. Auch können hierbei als Daten D Informationen über diagnostische und/oder therapeutische Mittel oder Ressourcen hinterlegt sein.

Jeder einzelne Behandlungsschritt 9 ist in einer Behandlungsfolge 10 abgespeichert. Die Behandlungsfolge 10 ist z. B. eine so genannte verkettete Liste, wobei jedes Listenelement durch einen Behandlungsschritt 9 gebildet wird. Mittels der Behandlungsfolge 10 bleiben auch in der Vergangenheit liegende Behandlungsschritte 9 anhand der Daten D dokumentierbar. Zudem ist die historische Entwicklung der Behandlung jederzeit nachvollziehbar. Die einfachste Form einer Behandlungsfolge 10 ist eine Behandlungsfolge 10 mit nur einem Behandlungsschritt 9. Dann sind Behandlungsfolge 10 und Behandlungsschritt 9 identisch.

Jeder Behandlungsschritt 9 umfasst als Daten D, also z. B. Symptome und Messwerte, die dem durch das jeweilige Ordnungsmerkmal 5 referenzierten Therapiehinweis 1 bzw. der Expertenregel 4 des Therapiehinweises 1 an dessen Eingängen 2 als Eingangsdaten E übermittelt werden. Die daraufhin von der Expertenregel 4 generierten individuellen Therapiehinweise 1, also z. B. Medikationen, werden als Ausgangsdaten A am Ausgang 3 des ersten Datensatzes D1 erzeugt und derart mit dem zweiten Datensatz D2 für die Therapieinformation 8 verknüpft, dass beispielsweise die Ausgangsdaten A im jeweiligen Behandlungsschritt 9 abgespeichert werden. Eingangs- und Ausgangsdaten E bzw. A können somit im jeweiligen Behandlungsschritt 9 entweder direkt oder indirekt, d. h. mittels einer Referenzierung des eigentlichen Ausgabe- oder Speicherortes, ausgegeben bzw. abgespeichert sein. Dadurch können, wenn z. B, in mehreren aufeinander folgenden Behandlungsschritten 9 immer wieder gleiche Ausgangsdaten A generiert werden oder immer wieder gleiche oder zumindest teilweise gleiche Eingangsdaten E aufgenommen werden. Mehrfacheintragungen werden verhindert, indem gleiche Eingangs- oder Ausgangsdaten E, A mehrfach einen Speicherplatz mit dem jeweils gleichen Eingangs- oder Ausgangsdatum E bzw. A referenzieren. Da je nach Grad und Umfang der Dokumentation der medizinischen Behandlung die Anzahl der zur Verfügung stehenden Eingangsdaten E und/oder die Anzahl der Ausgangsdaten A variieren kann, können zudem die Eingangs- und/oder die Ausgangsdaten E bzw. A beispielsweise in einer Eingangs- bzw. Ausgangsdatenliste (nicht dargestellt), insbesondere in Form einer verketteten Liste, abgelegt sein. Dies gestattet eine dynamische, bedarfsgerechte Bereitstellung von Speicherplatz für genau so viele Eingangsund Ausgangsdaten E bzw. A wie im konkreten Behandlungsschritt 9 zur Verfügung stehen bzw. generiert werden.

FIG 3 zeigt eine Variante der Therapieinformation 8 aus FIG 2. Die Variante der Therapieinformation 8 ist als Therapieinformation 8 mit einer Behandlungsfolge 10 mit nur einem Behandlungsschritt 9 dargestellt, d. h. die Therapieinformation 8 umfasst nur eine Gruppe von Eingängen E und nur eine Gruppe von Ausgängen A. Die Datenstruktur der Therapieinformation 8 wird in einer Darstellung wie in FIG 3 auf einer Benutzeroberfläche eines Arbeitsplatzrechners, also z. B. einem von einem Arzt in seinem Sprech- oder Behandlungszimmer verwendeten Personal Computer angezeigt. Die Eingangsdaten E wie Messdaten, z. B. EKG-Daten können entweder manuell oder mittels gebräuchlicher "drag and drop" Techniken in die Therapieinformation 8 eingetragen werden. Es ist auch möglich, dass anstelle der eigentlichen Eingangsdaten E eine Referenz auf den Speicherort der Eingangsdaten E eingetragen wird. Die mittels des Therapiehinweises 1 anhand der Expertenregeln 4 abgeleiteten Ausgangsdaten A werden im ersten Datensatz D1 markiert und zusätzlich optional (z. B. eine Diagnose) oder automatisch in die Therapieinformation 8 eingetragen. Bei einer Auswertung des der Therapieinformation 8 zugrunde liegenden Therapiehinweises 1 durch den Arzt oder Therapeuten erfolgt die Eintragung der Ausgangsdaten A aus dem ersten Datensatz D1 in den zweiten Datensatz D2 manuell. Bei elektronischer Implementierung der dem Therapiehinweis 1 zugrunde liegenden Expertenregel 4 kann der Eintrag der Ausgangsdaten A des ersten Datensatzes D1 in den zweiten Datensatz D2 selbst auch automatisch erfolgen.

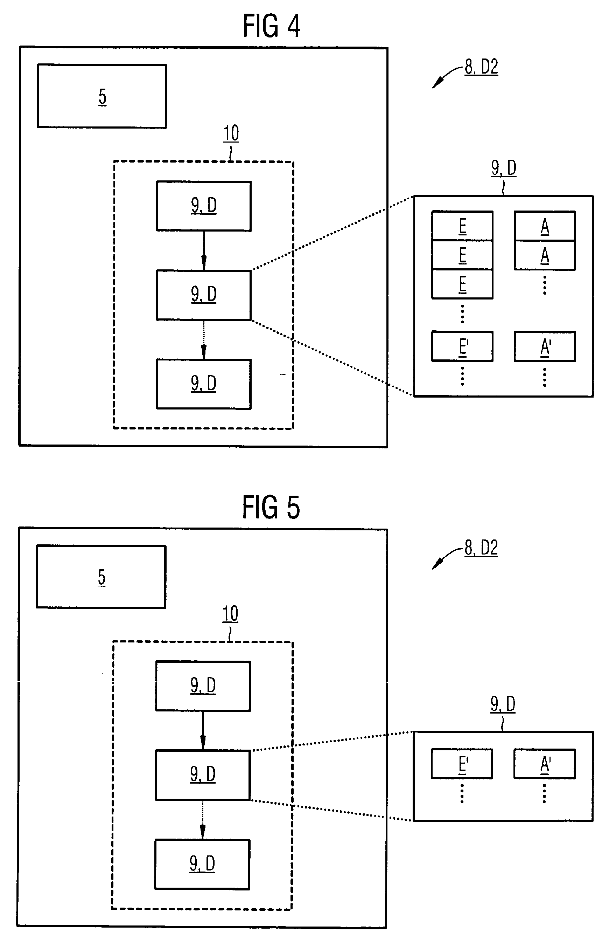

FIG 4 zeigt schematisch eine Therapieinformation 8. Die Therapieinformation 8 umfasst wie die Therapieinformation 8 aus FIG 2 Eingangs- und Ausgangsdaten E, A. Darüber hinaus ist für den jeweiligen Behandlungsschritt 9 eine Angabe von frei definierbaren Eingangsdaten E' sowie eine Angabe von frei definierbaren Ausgangsdaten A' vorgesehen, welche beispielsweise zur genaueren oder umfangreicheren Dokumentation hinzugefügt werden. Mittels der frei definierbaren Eingangs- und Ausgangsdaten E' bzw. A' kann der Arzt oder Therapeut z. B. Ausnahmefälle und zu diesen die jeweils erforderlichen zusätzlichen Eingangs- und Ausgangsdaten E bzw. A erfassen.

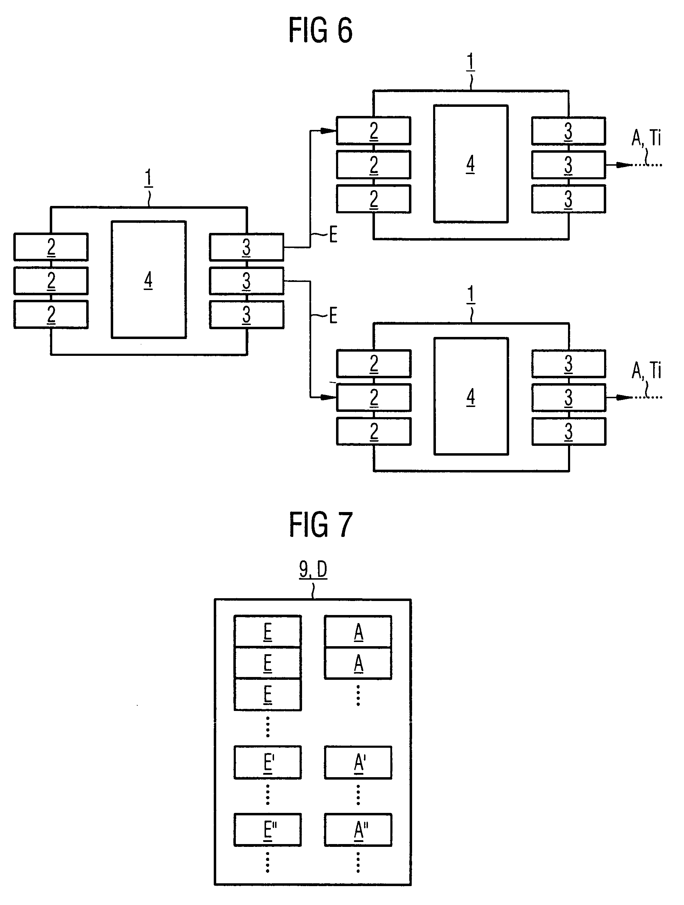

FIG 5 zeigt eine Therapieinformation 8 zur ausschließlichen Dokumentation von Ausnahmefällen. Die Therapieinformation 8 umfasst daher nur frei definierbare Eingangs- und Ausgangsdaten E', A'. Eine solche reduzierte oder minimierte Form der Therapieinformation 8 ist besonders dann geeignet, wenn der der Therapieinformation 8 zugrunde liegende Therapiehinweis 1 "trivial" ist, d. h. wenn sich die "normalen" Eingangs- und Ausgangsdaten E, A jederzeit aus dem zugrunde liegenden Therapiehinweis 1 selbst ableiten lassen.

FIG 6 zeigt kaskadierte Therapiehinweise 1. Solche kaskadierten, also verketteten Therapiehinweise 1 berücksichtigen, dass komplexe Therapiehinweise 1 oder medizinische Leitlinien aus einer Verkettung von mehreren Expertenregeln 4 bestehen. Die Verkettung ergibt sich dadurch, dass ein Ausgang 3 einer ersten Expertenregel 4 den Eingang 2 einer nachgeordneten weiteren Expertenregel 4 bildet.

FIG 7 zeigt eine Therapieinformation 8 für verkettete Therapiehinweise 1 eines Behandlungsschritts 9. Einzelne Eingangsund Ausgangsdaten E bzw. A sind als "primäre" Eingangs- und Ausgangsdaten E, A in Bezug auf den ersten Therapiehinweis 1 der verketteten Therapiehinweise 1 vorgesehen. Darüber hinaus sind frei definierbare Eingangs- und Ausgangsdaten E', A' vorgesehen, die einzelnen Therapiehinweisen 1 der verkettete Therapiehinweise 1 zugeordnet sind. Schließlich sind optionale Eingangs- und Ausgangsdaten E", A" vorgesehen, die für weitere Therapiehinweise 1 entlang der Verkettung der verketteten Therapiehinweise 1 erforderlich werden.

Die Eingangs- und Ausgangsdaten E, E" bzw. A, A" sowie die frei definierbaren Eingangs- und Ausgangsdaten E', A' werden gespeichert und archiviert, um die Möglichkeit zu nutzen, medizinische Institutionen, insbesondere solche Institutionen, die die Therapiehinweise 1 erstellen und/oder verwalten, über die Häufigkeit und die Art von Ausnahmefällen informieren zu können. Die auf diese Weise informierte Institution hat dann die Möglichkeit, die jeweiligen Therapiehinweise 1 anzupassen oder zu erweitern, sofern die Ausnahmefälle mit relevanter Häufigkeit auftreten.

Die Anpassung oder Erweiterung der Therapiehinweise 1 ist für die zuständige Institution dann besonders effizient und zielgerichtet durchführbar, wenn in den Therapiehinweisen 8 gespeicherte Daten, also die Eingangs- und Ausgangsdaten E, E" bzw. A, A" sowie die frei definierbaren Eingangs- und Ausgangsdaten E', A', an die Institution übermittelt werden. Die Daten D werden vorzugsweise in anonymisierter Form, d. h. ohne den Namen des Patienten, etc., weitergeleitet. Aus diesen Daten D kann, insbesondere wenn diese von einer Vielzahl von Institutionen geliefert werden, eine Statistik erstellt werden. Diese Statistik ermöglicht z. B. eine Aussage darüber, welche Ausgangsdaten A, A`, A" besonders häufig auftreten. Des Weiteren ermöglicht eine solche Statistik einen Vergleich der Häufigkeit von Therapieinformationen 8 ohne frei definierbare Eingangs- und Ausgangsdaten E, E", A, A" (vorgesehene Fälle) mit der Häufigkeit von Therapieinformationen mit frei definierbare Eingangs- und Ausgangsdaten E', A' (Ausnahmefälle). Anhand einer solchen Häufigkeitsanalyse lässt sich ein Maß für die Notwendigkeit einer Änderung oder Ergänzung eines Therapiehinweises 1 ableiten. Wenn die frei definierbaren Eingangs- und Ausgangsdaten E', A' an die zuständige Institution weitergeleitet wurden, können diese, oder zumindest diejenigen mit signifikanter Häufigkeit, in die Expertenregel 4 des zu ändernden oder zu ergänzenden Therapiehinweises 1 einbezogen werden.

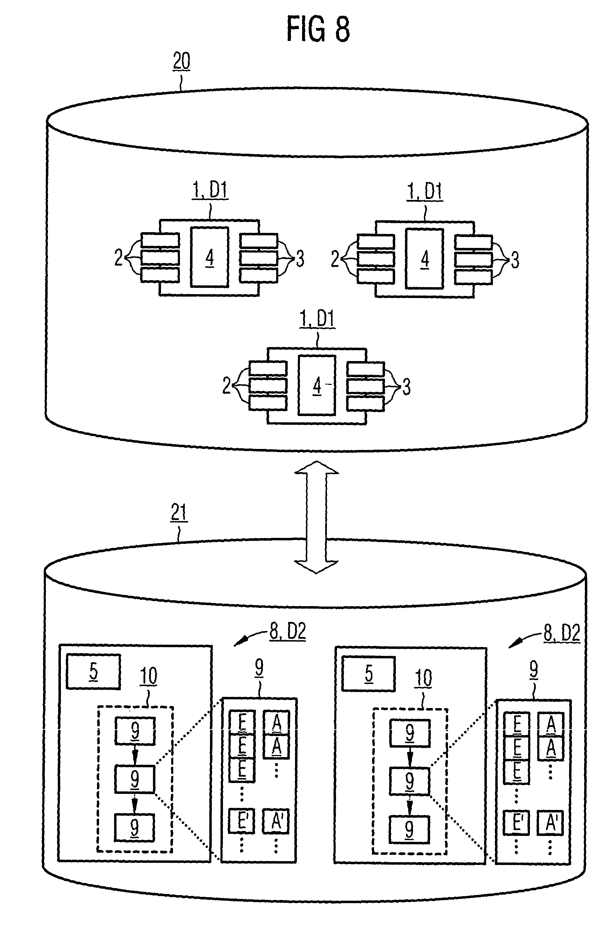

Diese Wechselwirkung zwischen den einzelnen Therapieinformationen 8 und den Therapiehinweisen 1, auf denen sie basieren, ist in FIG 8 schematisch dargestellt. Die Therapiehinweise 1 sind in einer zentralen Datenbank 20 mittels mehrerer erster Datensätze D1 gespeichert. Die zentrale Datenbank 20 wird von einer übergeordneten medizinischen Institution, z. B. von einem anerkannten Gremium der Ärzteschaft, betrieben. Die Therapieinformationen 8 sind mittels mehrerer zweiter Datensätze D2 in einer lokalen Datenbank 21 gespeichert. Diese wird von einer lokalen medizinischen Institution, also z. B. einer Klinik, für eine Arztpraxis etc., betrieben.

In der lokalen Datenbank 21 werden die einzelnen Therapieinformationen 8 mit der fortschreitenden Behandlung, die sie beschreiben, ereignis- oder zeitgesteuert aktualisiert. Der behandelnde Mediziner, also der Arzt oder Therapeut, trägt Eingangsdaten E, E" in die jeweilige Therapieinformation 8 ein. Bei automatischer Anwendung der Expertenregel 4 des zugrunde liegenden Therapiehinweises 1 werden die generierten Ausgangsdaten A, A" auch automatisch in die Therapieinformation 8 eingetragen. Wenn der Mediziner sich an den Therapiehinweis 1 hält, kann sich hier keine Inkonsistenz ergeben. Wenn die Expertenregel 4 durch den Mediziner selbst angewandt wird, trägt auch der Mediziner selbst die von ihm ermittelten Ausgangsdaten A, A" in die Therapieinformation 8 ein. Wenn eine Eingabe von Ausgangsdaten A, A" nur entsprechend einem vorgegebenen Format ausgeführt wird, d. h. eine automatische Auswertung der eingegebenen Ausgangsdaten A, A" möglich ist, können zu einem späteren Zeitpunkt die vom Mediziner ermittelten und eingegebenen Ausgangsdaten A, A" mit Ausgangsdaten A, A" vergleichen werden, die sich bei automatischer Anwendung der Expertenregel 4 ergeben hätten. Auf diese Weise können evtl. Behandlungsfehler des Mediziners, aber auch Unzulänglichkeiten der Expertenregel 4 erkannt werden. Wenn der Mediziner von den automatisch, zeit- oder ereignisgesteuert ermittelten Ausgangsdaten A, A" der Expertenregel 4 abweichen will, benutzt er bei der Dokumentation eines solchen Schrittes in der Therapieinformation 8 dafür vorgesehene frei definierbare Eingangs- und Ausgangsdaten E', A'. Diese frei definierbaren Eingangs- und Ausgangsdaten E', A' werden in der jeweiligen Therapieinformation 8 gespeichert und können an die die zentrale Datenbank 20 betreibende Institution zum Erkennen von evtl. Ergänzungsbedarf bei den Therapiehinweisen 1 herangezogen werden. Ein solcher Ergänzungsbedarf kann sich auf eine Überarbeitung oder Ergänzung einzelner Therapiehinweise 1, auf die Notwendigkeit einer Ersetzung eines (veralteten) Therapiehinweises 1 sowie auf die Erzeugung neuer, bisher noch nicht vorgesehener Therapiehinweise 1 etc. beziehen.

Bei der Überprüfung von zurückliegenden Diagnosestellungen und Therapieentscheidungen kommen übergeordnete Regelwerke, die nach Art und Struktur einem Therapiehinweis 1 entsprechen und deshalb auch nicht gesondert dargestellt sind, zum Einsatz. Dabei sind folgende Vorgehensweisen möglich: Ausgehend von einer in einer Therapieinformation 8 (Patientenakte) niedergelegten Diagnosestellung oder Therapieentscheidung wird das zugehörige Regelwerk (= Expertenregel 4), z. B. anhand von Codes oder anhand der Patientendaten ausgewählt. Daraufhin wird getestet, ob die Therapieinformation 8 die von dem Regelwerk geforderten Daten D aufweist. Je nach Ergebnis wird die Konformität der Diagnosestellung oder Therapieentscheidung (= individueller Therapiehinweis Ti) mit dem Regelwerk bescheinigt oder eine negative Bescheinigung ausgegeben. War die getroffene Diagnosestellung oder Therapieentscheidung nicht regelwerkskonform wird auf die nicht erfüllten Bedingungen des Regelwerks hingewiesen. Zusätzlich oder alternativ können anhand des jeweils zur Analyse herangezogenen Regelwerkes zusätzliche Untersuchungen, welche die Diagnosestellung oder Therapieentscheidung bestätigen könnte, vorgeschlagen werden. Des Weiteren, also nochmals zusätzlich oder alternativ kann auch anhand des jeweils zur Analyse herangezogenen Regelwerkes ein Hinweis auf Krankheitsbilder oder Symptomatiken ausgegeben werden, bei denen besonders häufig Verwechslungen vorkommen. Sind solche Verwechslungen denkbar, werden alternative Diagnosen, insbesondere mit einem Hinweise, welche zusätzlichen Untersuchungen dafür erforderlich sind, angegeben.

Die Überprüfung von zurückliegenden Diagnosestellungen und Therapieentscheidungen wird bevorzugt bei jeder neuen Diagnosestellung, nach jeder Überweisung an einen weiterbehandelnden Arzt, bei jedem neuen Eintrag in die Patientenakte, periodisch zu vorgegebenen oder vorgebbaren, insbesondere äquidistanten Zeitpunkten oder nach jeder Aktualisierung des Regelwerkes oder der Therapiehinweise und somit zeit- und/oder ereignisgesteuert durchgeführt.

## Patentansprüche

1. Verfahren zur Verarbeitung von Therapiehinweise (1) umfassenden Datensätzen (D) bei medizinischen Behandlungen, wobei ein erster Datensatz (D1) eines Therapiehinweises (1) mindestens einen Eingang (2) und mindestens einen Ausgang (3) sowie eine den Eingang (2) und den Ausgang (3) verknüpfende Expertenregel (4) umfasst, und ein Therapieinformationen (8) umfassender zweiter Datensatz (D2) mit Eingangsdaten (E) des ersten Datensatzes D1 des betreffenden Therapiehinweises (1) derart verknüpft wird, dass die Eingangsdaten (E) anhand für die medizinische Behandlung erforderlichen Parametern (P) der Therapieinformation (8) des zweiten Datensatzes (D2) bestimmt und dem ersten Datensatz (D1) zugeführt werden, wobei anhand der Eingangsdaten (E) mittels der Expertenregel (4) am Ausgang (3) des Datensatzes (D1) individuelle Therapiehinweise (Ti) in Form der Ausgangsdaten (A) erzeugt, ausgegeben und/oder hinterlegt werden.

2. Verfahren nach Anspruch 1, wobei die Eingangsdaten (E) ereignis- und/oder zeitgesteuert dem ersten Datensatz (D1) des Therapiehinweises (1) zugeführt wird.

3. Verfahren nach Anspruch 1 oder 2, wobei die Ausgangsdaten (A) automatisch oder ereignisbedingt ausgegeben werden.

4. Verfahren nach einem der vorhergehenden Ansprüche 1 bis 3, wobei dem Therapiehinweis (1) ein Ordnungsmerkmal (5) zugeordnet wird, anhand dessen der Therapiehinweis (1) eindeutig identifiziert wird.

5. Verfahren nach Anspruch 4, wobei das Ordnungsmerkmal (5) desjenigen Therapiehinweises (1), welcher der medizinischen Behandlung zugrunde liegt, der Therapieinformation (8) zugeordnet wird.

6. Verfahren nach Anspruch 4 oder 5, wobei dem Ordnungsmerkmal (5) die Eingangsdaten (E) und/oder die Ausgangsdaten (A) des zugrunde liegenden Therapiehinweises (1) zugeordnet werden.

7. Verfahren nach einem der Ansprüche 1 bis 6, wobei der Therapieinformation (8) mindestens ein optionales Eingangsdatum (E') und/oder mindestens ein optionales Ausgangsdatum (A') des zugrunde liegenden Therapiehinweises (1) zugeordnet wird.

8. Verfahren nach einem der Ansprüche 1 bis 7, wobei der Therapieinformation (8) mindestens ein weiteres frei definierbares Eingangsdatum (E") und/oder mindestens ein weiteres frei definierbares Ausgangsdatum (A") des Therapiehinweises (1) zugeordnet wird.

9. Verfahren nach einem der vorhergehenden Ansprüche 1 bis 8, wobei die vom Therapiehinweis (1) an dessen Ausgang (3) generierten individuellen Therapiehinweise (Ti) automatisch in die jeweiligen Ausgangsdaten (A, A', A") der zugehörigen Therapieinformation (8) übertragen werden.

10. Verfahren nach einem der vorhergehenden Ansprüche 1 bis 9, wobei eine Änderung einzelner der Therapieinformation (8) zugeordneten Ausgangsdaten (A, A', A") und/oder Eingangsdaten (E, E', E") gemäß einem vorgegebenen Format ausgeführt werden.

11. Verfahren nach Anspruch 10, wobei bei oder vor einer Änderung einzelner Eingangsdaten (E, E', E") und/oder Ausgangsdaten (A, A', A") der Therapieinformation (8) eine diesbezügliche Berechtigung überprüft wird.

12. Verfahren nach einem der vorhergehenden Ansprüche 1 bis 11, wobei die in einzelnen oder in einer Mehrzahl von Therapieinformationen (8) gespeicherten Eingangsdaten (E, E', E") und/oder Ausgangsdaten (A, A', A") zur Ergänzung und/oder Aktualisierung von Therapiehinweisen (1) verwendet werden.

13. Verfahren nach einem der vorhergehenden Ansprüche 1 bis 12, wobei der Therapiehinweis (1) in eine Mehrzahl von miteinander verketteten Therapiehinweisen (1) unterteilt wird und wobei das oder jedes Eingangsdatum (E) einem ersten Therapiehinweis (1) in der Kette der verketteten Therapiehinweise (1) zugeführt wird.

14. Verfahren nach Anspruch 13, wobei das oder jedes optionale Eingangsdatum (E') ein dem ersten Therapiehinweis (1) in der Kette der verketteten Therapiehinweise (1) nachfolgenden Therapiehinweis (1) zugeführt wird.

15. Verfahren nach Anspruch 13 oder 14, wobei anhand des Ausgangsdatums (A) des ersten Therapiehinweises (1) in der Kette der verketteten Therapiehinweise (1) an dessen mindestens einem Ausgang (3) individuelle Therapiehinweise (Ti) generiert und ausgegeben werden.

16. Verfahren nach einem der Ansprüche 13 bis 15, wobei anhand des optionalen Ausgangsdatums (A') eines dem ersten Therapiehinweis (1) in der Kette der verketteten Therapiehinweise (1) nachfolgenden Therapiehinweises (1) an dessen mindestens einem Ausgang (3) weitere individuelle Therapiehinweise (Ti) generiert und ausgegeben werden.

17. Verfahren nach einem der vorhergehenden Ansprüche 1 bis 16, wobei die in einzelnen oder in einer Mehrzahl von Therapieinformationen (8) gespeicherten Eingangsdaten (E, E', E") und/oder Ausgangsdaten (A, A', A") zur Überprüfung von Diagnosestellungen oder Therapieentscheidungen verwendet werden.

18. Verfahren nach einem der vorhergehenden Ansprüche 1 bis 17, wobei jeder Therapiehinweis (1) mindestens einen Behandlungsschritt (9) umfasst und wobei der Therapieinformation (8) eine Mehrzahl von Behandlungsschritten (9) in Form einer als verkettete Liste realisierten Behandlungsfolge (10) zugeordnet werden.
